(19) Europäisches Patentamt
European Patent Office
Office européen des brevets

(11) EP 1 511 726 B1

(12) # FASCICULE DE BREVET EUROPEEN

(45) Date de publication et mention de la délivrance du brevet:
**09.11.2005 Bulletin 2005/45**

(21) Numéro de dépôt: **03757119.7**

(22) Date de dépôt: **06.06.2003**

(51) Int Cl.[7]: **C07D 209/10**, C07C 211/49

(86) Numéro de dépôt international:
**PCT/FR2003/001703**

(87) Numéro de publication internationale:
**WO 2003/104194 (18.12.2003 Gazette 2003/51)**

(54) **PROCEDE DE PREPARATION DE 1,3,5-TRIAMINOBENZENE ET SON HYDROLYSE EN PHLOROGLUCINOL DE HAUTE PURETE**

VERFAHREN ZUR HERSTELLUNG VON 1,3,5-TRIAMINOBENZOL UND DESSEN HYDROLYSE ZU HOCHREINEM PHLOROGLUCINOL

METHOD FOR PREPARING 1,3,5-TRIAMINOBENZENE AND HYDROLYZING IT INTO HIGH-PURITY PHLOROGLUCINOL

(84) Etats contractants désignés:
**AT BE BG CH CY CZ DE DK EE ES FI FR GB GR HU IE IT LI LU MC NL PT RO SE SI SK TR**

(30) Priorité: **11.06.2002 FR 0207177**

(43) Date de publication de la demande:
**09.03.2005 Bulletin 2005/10**

(73) Titulaire: **PHV Analytic**
**39000 Lons le Saunier (FR)**

(72) Inventeurs:
- **ISMAILI, Lhassane**
  **F-25000 Besançon (FR)**
- **REFOUVELET, Bernard**
  **F-25000 Besançon (FR)**
- **XICLUNA, Alain**
  **F-39120 Annoire (FR)**

(74) Mandataire: **Doressamy, Clarisse**
**Cabinet Plasseraud**
**65/67 rue de la Victoire**
**75440 Paris Cedex 09 (FR)**

(56) Documents cités:
**EP-A- 0 643 962** **FR-A- 2 481 272**
**GB-A- 2 110 680** **US-A- 4 115 451**

- **DATABASE WPI Section Ch, Week 199050 Derwent Publications Ltd., London, GB; Class B05, AN 1990-372984 XP002226278 & JP 02 270831 A (MITSUI PETROCHEM IND CO LTD), 5 novembre 1990 (1990-11-05)**
- **DATABASE WPI Section Ch, Week 198342 Derwent Publications Ltd., London, GB; Class B05, AN 1983-791050 XP002226279 & JP 58 150530 A (MITSUI PETROCHEM IND CO LTD), 7 septembre 1983 (1983-09-07)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 511 726 B1

## Description

La présente invention concerne un procédé de préparation du 1,3,5-triaminobenzene et son hydrolyse puis purification en phloroglucinol de haute pureté.

Le phloroglucinol est un composé connu aussi bien du teinturier que du pharmacien. On s'intéressa en premier lieu au phloroglucinol pour son utilisation en teinture pour papiers ou textiles. Ce n'est que plus tard que les pharmaciens lui découvrirent des propriétés antispasmolytiques musculotropes. Il est cependant clair que les exigences de pureté sont beaucoup plus élevées lorsque le phloroglucinol est utilisé en tant qu'antispasmodique qu'en tant qu'agent de teinture.

La littérature décrit d'une façon extensive la préparation du phloroglucinol par l'hydrolyse du 1,3,5-triaminobenzene en présence d'acide chlorhydrique concentré. Le 1,3,5-triaminobenzène représente donc un intermédiaire très utilisé dans la préparation du phloroglucinol.

En ce qui concerne la préparation du 1,3,5-triaminobenzène, un nombre important de voies de synthèses ont déjà été proposées.

Parmi les voies de synthèses déjà proposées, on peut citer le brevet US 4,380,670. Ce brevet décrit la préparation de 1,3,5-triaminobenzène à partir du 3,5-diaminochlorobenzene en présence d'ammoniaque et de sels ou d'oxydes de cuivre à divers degrés d'oxydation à une température comprise entre 150 et 250°C. Ce brevet précise en outre en colonne 1, lignes 38 à 42 que la préparation du 1,3,5-triaminobenzène par amination directe du 1,3,5-trichlorobenzène n'est pas possible. Les auteurs du brevet indiquent clairement que la réaction d'amination désirée n'a pas lieu.

Une autre voie de synthèse possible du 1,3,5-triaminobenzène est décrite par H.T. Clarke et W. W. Hartman dans l'article intitulé "Phloroglucinol", Organic synthesis, vol. 45. Dans cet article, le 1,3,5-triaminobenzène est obtenu en partant de l'acide 2,4,6-trinitrobenzoique dans l'acide chlorhydrique concentré en présence d'étain. Toutefois la synthèse de l'acide trinitrobenzoïque est relativement longue et délicate, nécessitant la préparation du trinitrotoluène (TNT) qui est explosif. En outre, la préparation du 1,3,5-triaminobenzène en partant de l'acide trinitrobenzoïque engendre des difficultés de purification. En effet, après hydrolyse du 1,3,5-triaminobenzène, il est particulièrement difficile de purifier le phloroglucinol obtenu. En conséquence de quoi, un phloroglucinol de haute pureté, répondant aux exigences pharmaceutiques, ne peut pas être obtenu par cette voie.

En ce qui concerne plus précisément l'étape ultérieure d'hydrolyse du 1,3,5-triaminobenzène pour obtenir le phloroglucinol, on peut citer le brevet US 4,115,451. Ce brevet préconise une hydrolyse du 1,3,5-triaminobenzène dans un excès d'acide chlorhydrique concentré à une température de 100 à 200°C pour aboutir au phloroglucinol. Cette étape d'hydrolyse est suivie d'une étape d'extraction avec un ester acétique. La phase extraite contenant le phloroglucinol cristallise après refroidissement. Après filtration, le phloroglucinol est recristallisé dans de l'eau contenant du charbon actif.

Nonobstant toute cette littérature sur la synthèse du 1,3,5-triaminobenzène et sur l'hydrolyse en phloroglucinol, la préparation d'un phloroglucinol de haute pureté pose encore de nombreux problèmes aux industriels du domaine pharmaceutique. Les exigences de pureté imposées par la pharmacopée requièrent une méthode de synthèse qui livre un phloroglucinol conforme aux critères de pureté pharmaceutique.

D'autre part, l'amélioration des voies de synthèse, en particulier le prix de revient des matières premières et la réduction du nombre des étapes de synthèse, ont des répercutions favorables sur les coûts de fabrication d'un principe actif pharmaceutique.

C'est en travaillant en ce sens que les inventeurs ont réussi à mettre au point un procédé de préparation de 1,3,5-triaminobenzène puis de son hydrolyse en phloroglucinol qui est original, efficace et moins coûteux. Ce procédé permet en outre d'obtenir un phloroglucinol de haute pureté tout à fait conforme aux exigences pharmaceutiques.

D'une manière générale, l'invention a pour objet un procédé de préparation de 1,3,5-triaminobenzène, comprenant une étape a) d'amination d'un composé de formule (I) :

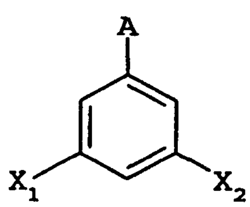

(I)

dans lequel :

A représente un atome d' halogène ou un groupe $NH_2$,

$X_1$ et $X_2$, qui sont identiques ou différents, représentent chacun un atome d'halogène,

ladite étape d'amination étant conduite en présence d'ammoniaque et d'un catalyseur choisi dans le groupe constitué par les sels de cuivre, les oxydes cuivrique et cuivreux et leurs mélanges, à une température allant de 150°C à 250°C et à une pression supérieure à 35 bars.

Il est à noter que le procédé selon l'invention est totalement original par rapport à l'art antérieur présenté ci-dessus.

En effet, comme il a été décrit plus haut, les inventeurs sont allés à l'encontre d'un préjugé technique du brevet US 4,380,670 et l'ont vaincu. Les inventeurs ont découvert contre toute attente qu'il est possible de réaliser l'amination du composé de formule (I), c'est-à-dire notamment du 1,3,5-trichlorobenzène ou de la 3,5-dichloroaniline, et d'obtenir le 1,3,5-triaminobenzène de façon quantitative, en une seule étape et à partir de composés stables et disponibles dans le commerce.

Dans la formule (I), A représente un groupe $NH_2$ ou un atome d'halogène, c'est-à-dire le brome, le chlore, le fluor ou encore l'iode. De préférence, A représente un groupe $NH_2$, le brome ou le chlore et plus préférentiellement le chlore.

$X_1$ et $X_2$ sont identiques ou différents l'un de l'autre et représentent un atome d'halogène, c'est-à-dire, comme indiqué ci-dessus, le brome, le chlore, le fluor ou encore l'iode, de préférence le chlore ou le brome.

D'une manière avantageuse, $X_1$ et $X_2$ sont identiques et représentent chacun un atome de brome ou de chlore, de préférence un atome de chlore.

Les composés (I) préférés sont le 1,3,5-trichlorobenzène, la 3,5-dichloroaniline, le 1,3,5-tribromobenzène ou la 3,5-dibromoaniline.

En ce qui concerne le catalyseur, celui-ci est de préférence choisi dans le groupe constitué par les sels halogénés de cuivre, encore appelés halogénures de cuivre, et de plus préférentiellement parmi le bromure de cuivre, le chlorure de cuivre, l'iodure de cuivre et leurs mélanges.

Ce catalyseur est de préférence utilisé dans des quantités allant de 1% à 5%, ce pourcentage exprimant le poids total de catalyseur sur le poids total de réactif.

Par ailleurs, cette étape a) est conduite en présence d'une solution d'ammoniaque dont la concentration est de préférence de 20 à 30%, et plus préférentiellement dont la concentration est égale à 28%.

Dans le procédé selon l'invention, cette solution d'ammoniaque est utilisée dans une quantité allant de préférence de 70% à 95% en poids sur le poids total des réactifs.

Le procédé selon l'invention peut également comprendre une étape supplémentaire d'hydrolyse du 1,3,5-triaminobenzène en phloroglucinol ainsi que des étapes éventuelles de purification de ce dernier.

Le procédé selon l'invention fournit d'ailleurs un 1,3,5-triaminobenzène particulièrement approprié pour une utilisation pour la préparation de phloroglucinol par hydrolyse.

Cette hydrolyse peut ainsi être réalisée de la façon suivante :

b) hydrolyse du 1,3,5-triaminobenzène obtenu à l'étape a) en présence d'acide chlorhydrique ou d'acide sulfurique à une température supérieure à 90°C, et de préférence de 100 à 120°C, pendant une durée de 6 à 24h pour obtenir un hydrolysat contenant du phloroglucinol,

c) éventuellement filtration à température ambiante de l'hydrolysat obtenu à l'étape b),

d) extraction du phloroglucinol de l'hydrolysat obtenu à l'étape b) ou du filtrat obtenu à l'étape c) par de l'éther éthylique ou un autre solvant à base ester, par exemple le benzoate d'éthyle, l'acétate d'éthyle, l'acétate d'isopropyle ou l'acétate de n-butyle.

Dans cette étape d'hydrolyse, l'acide chlorhydrique peut notamment être à une concentration de 20 à 40%, de préférence une concentration de 37% et dans des quantités allant de 10% à 15% en poids sur le poids total de réactif. L'acide sulfurique peut être à une concentration de 10%V à 100%V, de préférence de 50%V à 98%V, les quantités allant de 2 à 6 équivalents en $H^+$, de préférence 4 équivalents en $H^+$.

Il est possible d'emprunter plusieurs voies pour la purification du phloroglucinol.

Une de ces voies comprend l'étape suivante :

e1) recristallisation du phloroglucinol obtenu à l'étape d) dans de l'eau contenant du charbon actif pour obtenir un phloroglucinol de haute pureté.

Une autre voie comprend la succession des étapes suivantes :

e2) concentration de l'hydrolysat obtenu à l'étape b) ou de la solution de phloroglucinol obtenue à l'étape d) jusqu'à précipitation du phloroglucinol,

f2) filtration du précipité obtenu à l'étape e2),

g2) recristallisation du phloroglucinol obtenu à l'étape f2) dans de l'eau contenant du charbon actif,

h2) reprise du phloroglucinol recristallisé obtenu à l'étape g2) dans de l'éther éthylique contenant du charbon actif pour obtenir une solution de phloroglucinol,

i2) évaporation de la solution de phloroglucinol obtenue à l'étape h2) pour obtenir un phloroglucinol de haute pureté.

Dans ces étapes de purification, aussi bien le charbon actif que les solvants sont utilisés dans des quantités habituellement mises en oeuvre par l'homme du métier.

Ce procédé de purification implique l'utilisation de l'éther et permet d'isoler un phloroglucinol répondant aux exigences de la pharmacopée puisqu'il présente entre autres propriétés une coloration inférieure ou égale à JB 5.

Des analyses du contrôle de la pureté ont été réalisées selon des méthodes décrites dans la présente demande de brevet. Selon ces analyses, le phloroglucinol obtenu selon le procédé de l'invention présente, au total, moins de 0,5% d'impuretés, de préférence moins de 0,2% d'impuretés et plus préférentiellement encore moins de 0,1% d'impuretés en poids sur le poids total de phloroglucinol obtenu.

Les trois impuretés les plus caractéristiques et les plus largement représentées dans ce type de préparation de phloroglucinol sont la 3,5-dichloroaniline, le phloroglucide et le résorcinol. Or, il a été mesuré que le

phloroglucinol obtenu selon le procédé de l'invention ne présente pas plus de 0,1%, de préférence pas plus de 0,05% et plus préférentiellement pas plus de 0,01% de ces trois impuretés en poids sur le poids total de phloroglucinol obtenu.

De tels niveaux d'impuretés satisfont totalement les exigences requises par la pharmacopée française. En conséquence de quoi, le phloroglucinol obtenu par le procédé selon l'invention est tout à fait indiqué pour la préparation d'un médicament, en particulier pour le traitement de troubles liés aux spasmes musculaires ou pour le traitement de la douleur chez un mammifère.

METHODES UTILISEES POUR LES ANALYSES

A- IDENTIFICATION

Le phloroglucinol obtenu est contrôlé selon la monographie "Phloroglucinol" de la Pharmacopée Française, Xème édition, Juillet 1987.

Spectre infrarouge : 3211 $cm^{-1}$, 1624 $cm^{-1}$, 1506 $cm^{-1}$, 1419, 5 $cm^{-1}$, 1157,2 $cm^{-1}$, 1008,7 $cm^{-1}$, 813 $cm^{-1}$.

Spectre RMN $^1H$ à 300 MHz dans DMSOd6 : 5,8 ppm, (s, 3H, C-H) et 9,1 ppm (s, 3H, O-H) .

Spectre RMN $^{13}C$ à 300 MHz dans DMSOd6 : 95,9 (C-H); 159,6 (C-OH).

B- PURETE

Les impuretés recherchées sont principalement la 3,5-dichloroaniline, le phloroglucide issu de la dimérisation du phloroglucinol et le résorcinol.

La 3,5-dichloroaniline est présente dans le phloroglucinol produit lorsque le procédé selon l'invention passe par l'étape a). La 3,5-dichloroaniline est en effet un des réactif de cette étape. En revanche, le phloroglucide et le résorcinol sont présents dans le phloroglucinol, quelles que soient ses étapes de préparation.

En pratique, on utilise la chromatographie liquide haute performance pour rechercher ces impuretés. Les méthodes qui peuvent être utilisées sont notamment les suivantes :

1- Identification et dosage de la 3,5-dichloroaniline

1.a- Chromatographie liquide haute performance comparative :

Préparation des solutions :

- Eluant Acétonitrile -$H_3PO_4$ (85%) à 0,5 $g.l^{-1}$ d'eau ;
- Solution témoin ($T_1$) : dissoudre 20,0 mg de 3,5-dichloroaniline de référence dans 100 ml d'éluant (alcool à 96% ; acétonitrile, acide dilué) ;
- Type de colonne : colonne Agilent Interchim ZORBAX SB-CN (4,6 × 250 mm) 5 µm, maintenue à 35°C avec une détection à 220 nm et un débit de 1 $ml.min^{-1}$ ;
- Solution témoin ($T_2$) : diluer la solution témoin ($T_1$) au 1/100ème dans l'eau ;
- Solution essai (E) : dissoudre 200,0 mg de phloroglucinol à analyser dans 100 ml d'eau.

Technique :

Les techniques mises en oeuvre peuvent varier légèrement selon le matériel utilisé. A titre d'exemple la technique peut être la suivante :

- dans un chromatographe convenablement équipé et réglé, injecter exactement 10 µl de chacune des solutions témoin et essai.
- mesurer pour chacune des solutions, les aires des pics obtenus et leur temps de rétention. La 3,5-dichloroaniline présente un pic ayant un temps de rétention égal TR ≈ 6,4 min.

Calcul :

Soient :

$A_1$ : la valeur de l'aire du pic de 3,5-dichloroaniline obtenue pour la solution témoin (T2);

$A_2$ : la valeur de l'aire du pic de 3,5-dichloroaniline obtenue pour la solution essai (E).

La teneur en % de 3,5-dichloroaniline sera donnée par l'expression :

$$t = (A_2/A_1) \times 0,1$$

Expression du résultat :

La teneur en 3,5-dichloroaniline contenue dans le phloroglucinol ne doit pas être supérieure à 0,1%.

2- Identification et dosage du phloroglucide

2.a- Chromatographie liquide haute performance comparative :

- colonne : Agilent Interchim ZORBAX SB-CN (4,6 × 250 mm) 5 µm, maintenue à 35°C ;
- 1,5 $ml.min^{-1}$ - détection : 220 nm.

Préparation des solutions :

- Eluant : $H_3PO_4$ (85%) à 0,5 g.l$^{-1}$ d'eau ;
- Solution témoin ($T_1$) : dissoudre 20,0 mg de phloroglucide de référence dans 100 ml de méthanol ;
- Solution témoin ($T_2$) : diluer la solution témoin ($T_1$) au 1/100ème dans l'eau ;
- Solution essai (E) : dissoudre 200 mg de phloroglucinol à analyser dans 100 ml d'eau.

Technique :

Les techniques mises en oeuvre peuvent varier légèrement selon le matériel utilisé. A titre d'exemple la technique peut être la suivante :

- dans un chromatographe convenablement équipé et réglé, injecter exactement 10 µl de chacune des solutions témoin et essai.
- mesurer pour chacune des solutions, les aires des pics obtenus et leur temps de rétention. Le phloroglucide présente un pic ayant un temps de rétention égal $T_R \approx 12{,}6$ min et le résorcinol un pic chromatographique à $T_R \approx 7{,}0$ min.

Calcul :

Soient :

$A_1$ : la valeur de l'aire du pic d'une impureté obtenue pour la solution témoin ;

$A_2$ : la valeur de l'aire du pic d'une impureté obtenue pour la solution essai.

La teneur en % de phloroglucide sera donnée par l'expression :

$$t = (A_2/A_1) \times 0{,}1$$

Expression du résultat :

La teneur en phloroglucide contenue dans le phloroglucinol ne doit pas être supérieure à 0,1%.

L'invention va maintenant être décrite plus en détail grâce aux exemples qui suivent. Ces exemples ont pour vocation d'illustrer le procédé de l'invention sans pour autant le limiter à ces simples modes de réalisations.

EXEMPLE 1 : Préparation du 1,3,5-triaminobenzène à partir du 1,3,5-trichlorobenzene et son hydrolyse en phloroglucinol.

Dans une cuve sous pression on place 5 g (27,5 mmol) de 1,3,5-trichlorobenzene on ajoute 70 ml d'ammoniaque à 28% et 800 mg d'iodure de cuivre. Le mélange est chauffé à 180°C et a une pression de 40 bar pendant 24h. Après refroidissement on ajoute 40 g de glace pilée et 79 ml d'acide chlorhydrique concentré puis le mélange est chauffé à 120°C pendant 20h. Le contenu du ballon est filtré, Le filtrat est ensuite extrait avec 3x40 ml d'éther éthylique. La phase éthéré est ensuite séché puis évaporé, on obtient 1,4 g de phloroglucinol soit un rendement de 40%

EXEMPLE 2 : Préparation du 1,3,5-triaminobenzène à partir de la 3,5-dichloroaniline et son hydrolyse en phloroglucinol.

Dans une cuve sous pression on place 3 g (18,5 mmol) de la 3,5-dichloroaniline on ajoute 50 ml d'ammoniaque à 28% et 300 mg d'iodure de cuivre. Le mélange est chauffé à 180°C et a une pression de 40 bar pendant 24h. Après refroidissement on ajoute 30 g de glace pilée et une solution d'acide chlorhydrique concentré 37% jusqu'à pH=1, puis le mélange est chauffé à 120°C pendant 20h.

Le contenu du ballon est filtré. Le filtrat est ensuite extrait avec 3x40 ml d'éther éthylique séché puis évaporé. On obtient un rendement de phloroglucinol de l'ordre de 60%.

EXEMPLE 3 : Hydrolyse du 1,3,5-triaminobenzène en phloroglucinol et son extraction à l'éther éthylique.

2,2 g (18 mmol) du 1,3,5-triaminobenzène dans 150 ml d'une solution aqueuse d'acide chlorhydrique 2N sont chauffés à 100°C pendant 18h. Après refroidissement à température ambiante, la solution est filtrée. La phase aqueuse est ensuite extraite avec 3×40 ml d'éther éthylique. Les phases éthérés sont séchées sur sulfate de sodium, filtrées puis évaporées.

Le phloroglucinol obtenu est ensuite recristallisé dans 17 ml d'eau contenant 15 mg de charbon actif on obtient 1,5 g de phloroglucinol pur.

EXEMPLE 4 : Hydrolyse du 1,3,5-triaminobenzène en phloroglucinol et sa purification à l'éther éthylique.

5 g de 1,3,5-triaminobenzène dans 300 ml d'une solution aqueuse d'acide chlorhydrique 0,5 N sont chauffés à 120°C pendant 15 heures. Après refroidissement, la solution est concentrée jusqu'à précipitation du phloroglucinol. Le précipité filtré est recristallisé dans 40 ml d'eau avec du charbon actif. Le produit obtenu est ensuite repris par un minimum d'éther éthylique et chauffé 15 min. avec du charbon actif. Après évaporation on obtient 2,9 g de produit pur.

EXEMPLE 5 : Préparation du 1,3,5-triaminobenzène à partir de la 3,5-dichloroaniline, hydrolyse avec l'acide

chlorhydrique du 1,3,5-triaminobenzène en phloroglucinol.

Dans une cuve sous pression, on place 30 g (18,6 mmole) de. 3,5-dichloroaniline et 1,8 g d'iodure de cuivre dans 160 ml d'ammoniaque à 28%. L'ensemble est chauffé à 190°C et sous une pression de 40 bars pendant 24h. Le contenu de la cuve est versé dans 200 ml d'eau puis l'excès d'ammoniaque est éliminé. On ajoute alors 56 g d'acide chlorhydrique 10 N et on chauffe à 110°C pendant 20h. Après filtration, la solution est refroidie dans un bain de glace jusqu'à précipitation du phloroglucinol. Le précipité obtenu est ensuite recristallisé dans 400 ml d'un mélange eau-méthanol (95V-5V). Une deuxième recristallisation dans le même mélange permet d'obtenir 12,5 g de phloroglucinol pur.

<u>EXEMPLE 6 :</u> Préparation du 1,3,5-triaminobenzène à partir de la 3,5-dichloroaniline ; hydrolyse avec l'acide sulfurique du 1,3,5-triaminobenzène en phloroglucinol et purification.

Dans une cuve sous pression, on place 30 g (18,6 mmol) de 3,5-dichloroaniline et 1,5 g de chlorure de cuivre dans 160 ml d'ammoniaque à 28%. L'ensemble est chauffé à 190°C et sous une pression de 37 bars pendant 20 heures. Le contenu de la cuve est versé dans 200 ml d'eau puis l'excès d'ammoniaque est éliminé. On ajoute alors 37 g d'acide sulfurique 98% et on chauffe à 110°C pendant 20 heures. Après filtration, la solution est concentrée au tiers puis refroidie dans un bain de glace jusqu'à précipitation du phloroglucinol. Le précipité obtenu est ensuite recristallisé dans 350 ml d'un mélange eau-éthanol (93 V - 7 V). Une deuxième recristallisation dans de l'eau permet d'obtenir 13 g de phloroglucinol pur.

## Revendications

1. Procédé de préparation de 1,3,5-triaminobenzène, **caractérisé par le fait qu'**il comprend une étape a) d'amination d'un composé de formule (I) :

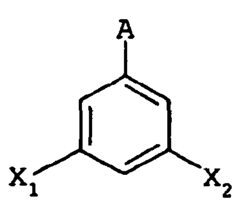

(I)

dans lequel :

A représente un atome d'halogène ou un groupe $NH_2$,
$X_1$ et $X_2$, qui sont identiques ou différents, représentent chacun un atome d'halogène,

ladite étape d'amination étant conduite en présence d'ammoniaque et d'un catalyseur choisi dans le groupe constitué par les sels de cuivre, les oxydes cuivrique et cuivreux et leurs mélanges, à une température allant de 150°C à 250°C et à une pression supérieure à 35 bars.

2. Procédé selon la revendication 1 selon lequel A représente un atome de brome, un atome de chlore ou un groupe $NH_2$, de préférence un atome de chlore ou un groupe $NH_2$ et plus préférentiellement un atome de chlore.

3. Procédé selon l'une ou l'autre des revendications 1 et 2 selon lequel $X_1$ et $X_2$ sont identiques et représentent chacun un atome de chlore ou un atome de brome, de préférence un atome de chlore.

4. Procédé selon l'une quelconque des revendications 1 à 3 selon lequel le catalyseur est choisi dans le groupe constitué par les halogénures de cuivre et les oxydes cuivrique et cuivreux, ledit catalyseur étant de préférence l'iodure de cuivre.

5. Procédé selon l'une quelconque des revendications 1 à 4 dans lequel l'ammoniaque possède une concentration de 20 à 30%, de préférence 28%.

6. Procédé selon l'une quelconque des revendications 1 à 5 comprenant en outre les étapes de :

b) hydrolyse du 1,3,5-triaminobenzène obtenu à l'issue de l'étape d'amination en présence d'acide chlorhydrique ou d'acide sulfurique à une température supérieure à 90°C, de préférence de 100 à 120°C pendant une durée de 6 à 24h pour obtenir un hydrolysat contenant du phloroglucinol,
c) éventuellement filtration à température ambiante de l'hydrolysat obtenu à l'étape b),
d) extraction du phloroglucinol de l'hydrolysat obtenu à l'étape b) ou du filtrat obtenu à l'étape c) par de l'éther éthylique ou un solvant à base ester, notamment par du benzoate d'éthyle, de l'acétate d'éthyle, de l'acétate d'isopropyle ou de l'acétate de n-butyle.

7. Procédé selon la revendication 6 dans lequel l'étape b) d'hydrolyse est conduite en présence d'acide chlorhydrique à une concentration de 20% à 40%, de préférence à une concentration de 37%.

8. Procédé selon la revendication 6 dans lequel l'étape b) d'hydrolyse est conduite en présence d'acide sulfurique à une concentration de 10%V à 100%V, de préférence de 50%V à 98%V.

9. Procédé selon la revendication 6 comprenant en

outre l'étape de :

e1) recristallisation du phloroglucinol obtenu à l'étape c) ou à l'étape d) dans de l'eau contenant du charbon actif pour obtenir un phloroglucinol de haute pureté.

**10.** Procédé selon la revendication 6 comprenant en outre les étapes de :

e2) concentration de l'hydrolysat obtenu à l'étape c) ou de la solution de phloroglucinol obtenue à l'étape d) jusqu'à précipitation du phloroglucinol,
f2) filtration du précipité obtenu à l'étape e2),
g2) recristallisation du phloroglucinol obtenu à l'étape f2) dans de l'eau contenant du charbon actif,
h2) reprise du phloroglucinol recristallisé obtenu à l'étape g2) dans de l'éther éthylique contenant du charbon actif pour obtenir une solution de phloroglucinol,
i2) évaporation de la solution de phloroglucinol obtenue à l'étape h2) pour obtenir un phloroglucinol de haute pureté.

## Patentansprüche

**1.** Verfahren zum Zubereiten von 1,3,5-Triaminobenzol, **dadurch gekennzeichnet, dass** es umfasst einen Schritt a) der Aminierung einer Verbindung der Formel (I):

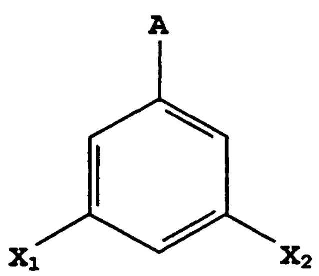

(I)

in der:

A ein Halogenatom oder eine $NH_2$-Gruppe repräsentiert,

$X_1$ und $X_2$, die identisch oder voneinander verschieden sind, jeweils ein Halogenatom repräsentieren,

wobei der besagte Aminierungsschritt bei einer Temperatur, die von 150 °C bis 250 °C reicht und bei einem Druck von mehr als 35 bar in Anwesenheit von Ammoniak und eines Katalysators durchgeführt wird, der aus der Gruppe ausgewählt ist, die von den Kupfersalzen, den Kupfer(II)oxiden und Kupfer(I)oxiden und deren Mischungen gebildet wird.

**2.** Verfahren nach Anspruch 1, bei dem A ein Bromatom, ein Chloratom oder eine $NH_2$-Gruppe repräsentiert, vorzugsweise ein Chloratom oder eine $NH_2$-Gruppe, und noch bevorzugter ein Chloratom.

**3.** Verfahren nach einem der Ansprüche 1 oder 2, bei dem $X_1$ und $X_2$ identisch sind und jedes ein Chloratom oder ein Bromatom, vorzugsweise ein Chloratom repräsentiert.

**4.** Verfahren nach einem der Ansprüche 1 bis 3, bei dem der Katalysator aus der Gruppe der Kupferhalogenide und der Kupfer(II)oxide und Kupfer(I)oxide ausgewählt ist, wobei der besagte Katalysator vorzugsweise Kupferjodid ist.

**5.** Verfahren nach einem der Ansprüche 1 bis 4, bei dem das Ammoniak eine Konzentration von 20 bis 30 %, vorzugsweise 28 % hat.

**6.** Verfahren nach einem der Ansprüche 1 bis 5, das außerdem die Schritte umfasst:

b) Hydrolyse des am Ende des Aminierungsschrittes erhaltenen 1,3,5-Triaminobenzols in Anwesenheit von Salzsäure oder Schwefelsäure bei einer Temperatur von mehr als 90 °C, vorzugsweise von 100 bis 120 °C, während einer Dauer von 6 bis 24 Stunden, um ein Phloroglucinol enthaltendes Hydrolysat zu erhalten,

c) eventuell Filtrierung des im Schritt b) erhaltenen Hydrolysates bei Umgebungstemperatur,

d) Extraktion des Phloroglucinols aus dem im Schritt b) erhaltenen Hydrolysat oder aus dem im Schritt c) erhaltenen Filtrat mittels Äthyläther oder eines Lösungsmittels auf Esterbasis, insbesondere mittels Äthylbenzoat, Äthylazetat, Isopropylazetat oder n-Butyl-Azetat.

**7.** Verfahren nach Anspruch 6, in dem der Hydrolyseschritt b) in Anwesenheit von Salzsäure bei einer Konzentration von 20 % bis 40 %, vorzugsweise bei einer Konzentration von 37 %, durchgeführt wird.

**8.** Verfahren nach Anspruch 6, in dem der Hydrolyseschritt b) in Anwesenheit von Schwefelsäure bei einer Konzentration von 10 %V bis 100 %V, vorzugsweise von 50 %V bis 98 %V, durchgeführt wird.

**9.** Verfahren nach Anspruch 6, das außerdem umfasst den Schritt:

e1) Rekristallisation des im Schritt c) oder im Schritt d) erhaltenen Phloroglucinols in Aktivkohle enthaltendem Wasser, um ein Phloroglucinol von hoher Reinheit zu erhalten.

**10.** Verfahren nach Anspruch 6, das außerdem umfasst die Schritte:

e2) Konzentrieren des im Schritt c) erhaltenen Hydrolysates oder der im Schritt d) erhaltenen Phloroglucinol-Lösung bis zum Ausfällen des Phloroglucinols,

f2) Filtrieren des im Schritt e2) erhaltenen Ausfällprodukts,

g2) Rekristallisation des im Schritt f2) erhaltenen Phloroglucinols in Aktivkohle enthaltendem Wasser,

h2) Wiederauflösen des im Schritt g2) erhaltenen rekristallisierten Phloroglucinols in Aktivkohle enthaltendem Äthyläther, um eine Phloroglucinol-Lösung zu erhalten,

i2) Eindampfen der im Schritt h2) erhaltenen Phloroglucinol-Lösung, um ein Phloroglucinol von hoher Reinheit zu erhalten.

## Claims

**1.** A method of preparing 1,3,5-triaminobenzene, **characterized in that** it comprises a step a) of amination of a compound of formula (I):

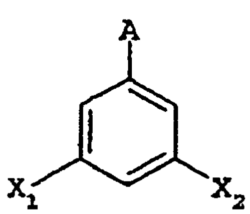

(I)

in which:

A represents a halogen atom or an $NH_2$ group, $X_1$ and $X_2$, which are identical or different, each represent a halogen atom,

said amination step being conducted in the presence of ammonia and a catalyst selected from the group consisting of copper salts, cupric and cuprous oxides and mixtures thereof, at a temperature ranging from 150°C to 250°C and at a pressure of greater than 35 bar.

**2.** The method of claim 1, wherein A represents a bromine atom, a chlorine atom or $NH_2$ group, preferably a chlorine atom or $NH_2$ group and more preferably a chlorine atom.

**3.** The method of one or the other of claims 1 and 2, wherein $X_1$ and $X_2$ are identical and each represent a chlorine atom or a bromine atom, preferably a chlorine atom.

**4.** The method of any one of claims 1 to 3, wherein the catalyst is selected from the group consisting of copper halides and cupric and cuprous oxides, said catalyst preferably being copper iodide.

**5.** The method of any one of claims 1 to 4, wherein the aqueous ammonia possesses a concentration of 20% to 30%, preferably 28%.

**6.** The method of any one of claims 1 to 5, further comprising the steps of:

b) hydrolysis of the 1,3,5-triaminobenzene obtained at the end of the amination step in the presence of hydrochloric acid or of sulfuric acid at a temperature greater than 90°C, and preferably from 100 to 120°C, for a time of 6 to 24 h, to give a hydrolysate containing phloroglucinol,
c) optionally filtration at ambient temperature of the hydrolysate obtained in step b),
d) extraction of phloroglucinol from the hydrolysate obtained in step b) or from the filtrate obtained in step c), using ethyl ether or an ester-based solvent, in particular with ethyl benzoate, ethyl acetate, isopropyl acetate or n-butyl acetate.

**7.** The method of claim 6, wherein step b) of hydrolysis is conducted in the presence of hydrochloric acid at a concentration of 20% to 40%, preferably at a concentration of 37%.

**8.** The method of claim 6, wherein step b) of hydrolysis is conducted in the presence of sulfuric acid at a concentration of 10% V to 100% V, preferably from 50% V to 98% V.

**9.** The method of claim 6, further comprising the step of:

e1) recrystallization of the phloroglucinol obtained in step c) or step d) from water containing active carbon, to give a high-purity phloroglucinol.

**10.** The method of claim 6, further comprising the steps of:

e2) concentration of the hydrolysate obtained in step c) or of the phloroglucinol solution obtained in step d) until phloroglucinol precipitates,
f2) filtration of the precipitate obtained in step e2),
g2) recrystallization of the phloroglucinol obtained in step f2) from water containing active carbon,
h2) takeup of the recrystallized phloroglucinol obtained in step g2) in ethyl ether containing active carbon, to give a phloroglucinol solution,
i2) evaporation of the phloroglucinol solution obtained in step h2), to give a high-purity phloroglucinol.